# EUROPEAN PATENT APPLICATION

(11) **EP 4 781 928 A1**
(43) Date of publication of application: **29.07.2026**
(21) Application number: 24867474.9
(22) Date of filing: 19.09.2024
(51) Int. Cl.: A61B 17/34

(54) **IMPLANT DELIVERY SYSTEM, CARDIAC RHYTHM ADJUSTMENT SYSTEM, AND CARDIAC RHYTHM ADJUSTMENT METHOD**

(30) Priority: 22.09.2023 CN 202311229455
(71) Applicant: Venus MedTech (HangZhou), Inc., Hangzhou, Zhejiang 310052 (CN)
(72) Inventor: CHEN, Mao, Hangzhou, Zhejiang 310052 (CN); XU, Yuanning, Hangzhou, Zhejiang 310052 (CN); ZENG, Xiaotong, Hangzhou, Zhejiang 310052 (CN)
(74) Representative: KIPA AB
(86) International application number: PCT/CN2024/119557
(87) International publication number: WO 2025/061068

(57) **Abstract**

Disclosed are an implant delivery system, a cardiac rhythm adjustment system, and a cardiac rhythm adjustment method. The implant delivery system includes a sheath (20) and an implant. The sheath (20) includes a hollow sheath body (12), and the implant is releasably disposed within the sheath body (12). A distal end of the sheath body (12) is a puncture tip (13) which can penetrate into a ventricular myocardium and guide the sheath body (12) to pass through the ventricular myocardium. A length of the sheath body (12) at least is sufficient to extend from outside the body, via a cardiac apex (4), into the ventricular myocardium. The implant is an implantable electrode (60, 61, 62, 63, 64, 65, 66), which is implanted via an intramyocardial pathway, so as to achieve real physiological position pacing and biventricular electrical synchronization by means of program control of a pulse generator (80).

## Description

### TECHNICAL FIELD

This application relates to the technical field of medical devices, and in particular to an implant delivery system, a cardiac rhythm adjustment system, and a cardiac rhythm adjustment method.

### BACKGROUND

In the prior art, implants are generally delivered into the human myocardium via endocardium, such as the delivery of hydrogel, drugs and electrodes. The implantable cardiac devices for treatment include conventional pacemakers, implantable cardioverter defibrillator (ICD), and cardiac resynchronization therapy(CRT)/cardiac resynchronization therapy-defbrilltor(CRT-D), which are common treatment manners for bradycardia, prevention and treatment of malignant ventricular arrhythmias, and severe heart failure with left bundle branch block, respectively. At present, the above-mentioned devices are mainly implanted via endocardium. An electrode wire of the left ventricular of the CRT/CRT-D mainly reaches the epicardium of the left ventricle via the coronary vein system.

The conventional pacemaker pacing the heart through the endocardium is not physiological pacing. After the electrode wire of the pacemaker is fixed to a surface of the endocardium, an initial electrical signal impulse directly excites the myocardial cells at the location of the electrode wire through the electrode, causing local myocardial cells to be excited in advance. At the same time, the electrical signal is transmitted through a signal path between myocardial cells and spreads to the surrounding area, causing excitation of myocardial cells in other parts and producing cardiac contraction. This pacing manner has a simple, fast, and reliable implantation manner, which can obtain stable pacing thresholds and effectively treat bradycardia. However, it changes the normal electrophysiological excitation sequence of the heart, leading to atrioventricular electrical asynchrony and biventricular electrical asynchrony, which in turn causes mechanical contraction of the heart to lose synchronization. Numerous clinical studies have confirmed that excessive right ventricular pacing increases the incidence of atrial fibrillation and heart failure, affecting the long-term prognosis of patients. Although His Bundle Pacing has become a new hotspot in recent years, due to the unique anatomy of the His Bundle and its surrounding structures, it has high operational difficulty, and its long-term efficacy and stability are still uncertain.

For ICD, there is a higher risk of electrode wire related complications. In addition to the pacing function for bradycardia, ICD will trigger electrical therapy based on the programmed parameters when detecting life-threatening malignant arrhythmia events, wherein the treatment methods include anti-tachycardia pacing, low-energy electrical cardioversion, and high-energy defibrillation. The biggest difference between the S-ICD/D-ICD and a regular pacemaker is the need of special electrode for the endocardium of the right ventricle. The right ventricular defibrillation electrode needs to withstand higher instantaneous high-energy discharges and is usually thicker and harder than ordinary right ventricular pacing electrodes. The incidence of complications related to right ventricular wire implantation in ICD is much higher than that of ordinary right ventricular pacing wires, including electrode breakage, insulation layer damage, vascular injury, etc. Once there is an abnormality in the defibrillation wire, it may lead to serious consequences such as ICD false discharge, non discharge, etc.

For CRT, its efficacy is influenced by various factors. According to literature reports, about 30% of heart failure patients receiving CRT treatment have no response or poor response to CRT treatment. During the CRT implantation process, the left ventricular electrode needs to be implanted to the epicardium of the left ventricular through the coronary vein system. The ideal implantation site for the left ventricular electrode is the latest activation point of the left ventricle. Whether the left ventricular electrode can be implanted in this ideal position highly depends on the course of the coronary vein and the morphology of its branches. It is usually recommended to choose the lateral vein or posterior vein of the coronary vein as the target vessel for implanting left ventricular electrodes. Once there are severe variations in the anatomy of the coronary vein, poor vascular conditions, or limitations such as phrenic nerve stimulation in patients, and the electrode wire of the left ventricular cannot be directly implanted to the optimal target vessel through the coronary vein, the response rate to CRT treatment will decrease.

### SUMMARY

### Technical problem

Although guidelines suggest that in this case, cardiac surgery (thoracoscopy) can be performed to directly fix the left ventricular electrode to the epicardium of the left ventricular target vascular area under direct visualization, there are still many difficulties and limitations in choosing this method in clinical practice. In recent years, there have also been literature reports that such patients can achieve CRT/CRT-D by direct pacing of the left ventricular endocardium through transatrial septal/interventricular puncture. However, it should be noted that patients undergoing direct pacing of the left ventricular endocardium require long-term oral anticoagulant therapy to prevent left ventricular system thrombosis.

### Technical solutions

In order to solve the above technical problems, the present application provides an implant delivery system, including a sheath and an implant, wherein
the sheath includes a hollow sheath body, the implant is releasably disposed within the sheath body, a distal end of the sheath body is a puncture tip capable of penetrating into a ventricular myocardium and guiding the sheath body to pass through the ventricular myocardium, and a length of the sheath body at least is sufficient to extend from outside a body, via a cardiac apex, into the ventricular myocardium.

Preferably, the implant is hydrogels or therapeutic drugs.

Preferably, a pushing device is slidably set inside the sheath body, and a distal end of the pushing device is capable of pushing the implant outside the sheath body for releasing.

Preferably, the pushing device is tubular-shaped and has an internal threading channel, a proximal end of the implant is fixed with a connecting wire or provided with a detachable connecting wire, and the connecting wire extends through the threading channel towards a proximal end.

Preferably, an internal of the sheath body includes a first lumen and a second lumen that extend in parallel from a distal end to a proximal end, and the implant includes multiple implants arranged along a length direction inside the first lumen;
a separating portion is provided between the first lumen and the second lumen, and multiple avoidance holes are defined in the separating portion; at least one of the avoidance holes is provided at a proximal end side of each implant, a connecting wire of each implant extend from a corresponding avoidance hole to the second lumen and then out of the proximal end of the sheath body.

Preferably, damping structures acting with each other are provided between the first lumen and each implant.

Preferably, a tubular-shaped pushing device is provided inside the second lumen for receiving the connecting wire.

Preferably, the pushing device can move along the connecting wire to enter the first lumen through the corresponding avoidance hole to push the corresponding implant. Preferably, the connecting wire is a conducting wire or a traction wire.

Preferably, there are multiple lumens defined in the sheath body and arranged in parallel, and there are multiple implants set in different lumens;
a pushing device is slidably set inside the sheath body, and a distal end of the pushing device is capable of pushing the implant outside the sheath body for releasing.

Preferably, the implant is an implantable electrode, and the system further includes a pulse generator, and wherein the implantable electrode and the pulse generator are formed separately, and the implantable electrode has a smooth outer surface; or
the implantable electrode and the pulse generator are formed integrally and have a smooth outer surface.

Preferably, the implant is an implantable electrode, and the system further includes a pulse generator, the implantable electrode has an anchor configured for acting on surrounding tissues, and the implantable electrode and the pulse generator are formed separately or integrally.

Preferably, a steering wire extends movably through the sheath body, a distal end of the steering wire is connected to an inner wall of the puncture tip, and a proximal end of the steering wire extends out of the sheath body and acts as a force application end for pulling the puncture tip to change its orientation.

Preferably, the implant delivery system further includes a guiding device, wherein the guiding device includes a base that is capable of being fixed to a body surface and an adjustable cooperating seat installed on the base, and the sheath is assembled to the cooperating seat.

Preferably, an arc-shaped guide rail is fixed on the base, the cooperating seat is installed on the arc-shaped guide rail in a slidable and positioning manner, and the sheath extends through the cooperating seat in a movable and positioning manner.

Preferably, the implant delivery system further includes an imaging system used to guide the sheath to puncture and display a position of the implant;
an ultrasound probe is provided on the cooperating seat to collect the position of the implant and a posture of the sheath in real-time, which is simulated and displayed through the imaging system;
wherein the position of the implant and the posture of the sheath are displayed at a corresponding position of a pre-constructed heart model, for comparison with a predetermined implantation site.

Preferably, an implantation site of the implant is predetermined electrical stimulation positions located in at least one of an interventricular septum myocardium, a left ventricular free wall myocardium, and a right ventricular free wall myocardium, one of the predetermined electrical stimulation positions is close to distal branches of a His bundle, or one of the predetermined electrical stimulation positions is close to the left bundle branch or the right bundle branch.

Meanwhile, the present application also provides a cardiac rhythm adjustment system, including:
at least one electrode being capable of being implanted into a ventricular myocardium;
a pulse generator connected to the electrode through a circuit, wherein the electrode and the pulse generator are formed separately or integrally; and
a sheath including a hollow sheath body, wherein the at least one electrode is releasably disposed within the sheath body, a distal end of the sheath body is a puncture tip capable of penetrating into the ventricular myocardium and guiding the sheath body to pass through the ventricular myocardium, and a length of the sheath body at least is sufficient to extend from outside a body, via a cardiac apex, into the ventricular myocardium.

In this application, the implanted electrode is located within the ventricular myocardium in its working state. Electrical stimulation is applied to corresponding position (i.e., electrical stimulation position) within the ventricular myocardium by inputting a specified electrical signal to the electrode, in order to intervene or adjust heart rhythm. In terms of working principle and circuit driving method, conventional technology can be used.

There is no strict limit on the number of the electrodes, such as one or multiple, which can be arranged at corresponding positions within the ventricular myocardium as needed.

Preferably, the electrode enters the ventricular myocardium through an epicardium and is located between the epicardium and the endocardium.

That is, the electrode has an implantation pathway from the epicardium to the ventricular myocardium, and is located between the epicardium and the endocardium in the working state.

In this application, a preferred manner for the electrode to enter the ventricular myocardium is through the epicardium, rather than the endocardium. That is, the electrode has an implantation pathway from the epicardium to the ventricular myocardium, and after entering the ventricular myocardium, it should be avoided from penetrating from the endocardium side. That is, the electrode itself is located between the epicardium and the endocardium. If electrical signal is input through wired connection, the conducting wire extends through the epicardium to the electrode inside the ventricular myocardium.

Preferably, the electrodes enter the ventricular myocardium through the body surface and the epicardium in sequence.

A preferred implantation pathway for the electrode to come into contact with and pass through the epicardium is from outside the body, such as implantation between the ribs under the guidance of the sheath.

Preferably, the electrode enters the ventricular myocardium through the epicardium at the cardiac apex.

The nerves at the cardiac apex are relatively sparse, and the implantation of the electrode through puncture into the epicardium from this location has little impact on the heart. The implantation pathway from the epicardium at the cardiac apex into the ventricular myocardium can avoid causing excessive stimulation to the heart during operation.

Preferably, the electrode enters the interventricular septum through the epicardium at the cardiac apex.

The ventricular myocardium generally includes an interventricular septum located between the left ventricle and right ventricle, as well as free wall myocardium located around each ventricle. The cardiac apex generally corresponds to the position of the interventricular septum myocardium, and it is convenient to enter the interventricular septum myocardium from the epicardium at the cardiac apex. In addition, the electrode can be closer to the end branches of the His bundle, which can influence the heart rhythm upstream of nerve signal transmission.

Preferably, the electrode enters the ventricular myocardium through the epicardium at the free wall of the ventricle.

Preferably, the electrode enters the free wall myocardium through the epicardium at the ventricular free wall.

When the location where the electrode needs to be implanted is at the ventricular free wall, a shorter implantation pathway can be chosen by puncturing the epicardium near the electrical stimulation position at the ventricular free wall.

Whether entering the epicardium through the cardiac apex or the free wall of the ventricle, it is advisable to avoid touching the His bundle and downstream branches as much as possible to improve the safety of implantation procedures.

Preferably, further including a reference electrode that can be placed in the right atrium to collect electrocardiogram signals.

In order to facilitate the collection of electrocardiogram signals to guide the timing and intensity of electrode electrical stimulation, reference electrodes can also be used to collect electrocardiogram signals. The method and pathway of placing the reference electrode in the right atrium are also described in the existing technology. In a preferred solution of this application, the implantation pathway of the reference electrode is improved, and it can also adopt a similar implantation pathway to the electrode, simplifying the operation steps.

Unless otherwise specified in this application, the electrode referred to herein refers to an electrode used for applying electrical stimulation and located within the ventricular myocardium. The position of the electrode is after implantation, i.e., in the working state.

Preferably, the reference electrode is connected to a pulse generator that receives signals collected by the reference electrode in a wireless manner.

Preferably, the reference electrode is connected to a pulse generator that receives signals collected by the reference electrode in a wired manner.

After implantation, the reference electrode can communicate with the pulse generator through wired or wireless means. In this application, whether the electrode or the reference electrode is wired, the connection and insulation methods of its wires can be based on existing technology; and when using wireless methods, the selected communication module and power supply can all adopt existing technologies, which is not the focus of improvement in this application.

Preferably, the reference electrode penetrates through the interventricular septum myocardium into the right atrium.

In order to simplify the operation, especially when the electrode is also located in the interventricular septum myocardium, the implantation of the reference electrode and the electrode can be completed through a one-time puncture operation. At least when switching the implantation object, the sheath does not need to completely withdraw from the epicardium, avoiding the adverse effects caused by multiple punctures.

In working state, a specific position of the electrode within the ventricular myocardium can be selected from multiple locations as needed.

Preferably, an implantation site of the electrode is predetermined electrical stimulation positions located in at least one of an interventricular septum myocardium, a left ventricular free wall myocardium, and a right ventricular free wall myocardium, one of the predetermined electrical stimulation positions is close to distal branches of a His bundle, or one of the predetermined electrical stimulation positions is close to the left bundle branch or the right bundle branch.

Preferably, the electrode is located within the interventricular septum myocardium.

Preferably, the free wall myocardium is left and/or right ventricular free wall myocardium.

Preferably, the electrode is located within the interventricular septum myocardium and/or the ventricular free wall myocardium.

When there is only one electrode, it can be located in the interventricular septum myocardium, left ventricular free wall myocardium, or right ventricular free wall myocardium. When there are multiple electrodes, they can be arranged in one of the three locations mentioned above, or distributed in two or three locations.

When the electrode is located within the interventricular septum myocardium, its specific location may be:
one of the electrode is preferably close to distal branches of the His bundle;
one of the electrode is preferably close to the left bundle branch or the right bundle branch.

In order to ensure the effectiveness of heart rhythm adjustment, in a preferred solution, the position of the electrode in the working state is close to distal branches of the His bundle or the left or right bundle branch. When using multiple electrodes, it can be understood that at least one electrode is located in one of the above positions.

Preferably, the electrode is located at a midpoint between the cardiac apex and the end branches of the His bundle along the long axis of the interventricular septum; or, slightly adjacent to the end branches of the His bundle; or, slightly adjacent to the cardiac apex.

The left and right bundle branches intersect at the end branches of the His bundle, and there is a relatively sparse area of nerves between the left and right bundle branches in the interventricular septum myocardium, which can be implanted with one or more electrodes. Electrodes can generally be divided into one or multiple groups based on their wire extension and splitting. Within the same group of electrodes, the electrode wires are bundled together, for example, by arranging all wires side by side or nested inside and outside, to facilitate one-time implantation of electrodes in the same group.

Preferably, there are one or multiple groups of electrodes in the interventricular septum myocardium, with each group distributed along the short axis of the interventricular septum.

Preferably, there should be one or multiple electrodes in the same group, distributed along the long axis of the interventricular septum.

Preferably, there are one or multiple groups of electrodes within the myocardium of the ventricular free wall, with each group distributed at intervals along the circumference of the ventricular free wall.

Preferably, there should be one or multiple electrodes in the same group, distributed along the long axis of the ventricular free wall.

Due to the irregular shape of the interventricular septum, the arrangement direction of different groups should be understood as a rough trend. The long and short axes of the interventricular septum are relative, and the long axis of the interventricular septum is roughly parallel to the long axis of the ventricular free wall. Generally, the longitudinal direction is often considered as the long axis.

Preferably, the electrode in the interventricular septum myocardium is pacing electrode.

Preferably, the electrode in the ventricular free wall myocardium is pacing electrode and/or defibrillation electrode.

Preferably, there are one or multiple electrodes, all of which have the same polarity, or two adjacent electrodes in a pair with opposite polarity.

Preferably, there are one or multiple groups of electrodes, each group has one or multiple electrodes, and the electrodes in the same group correspond to an arrangement path within the ventricular myocardium.

Preferably, the arrangement path roughly extends along a straight line.

When there are a large number of electrodes, they can be divided into multiple groups according to their distribution position or wire bundle situation. In the wording state, the spatial connection or extension trend of the electrodes in the same group can be regarded as the arrangement path. This arrangement path may be a virtual spatial position relationship. In the case of wired connection, it can also be regarded as the direction of the conducting wires of the electrodes in the same group passing through the ventricular myocardium.

Preferably, the arrangement paths within the ventricular myocardium are distributed radially around a cardiac apex.

Preferably, the arrangement paths within the ventricular myocardium are parallel to each other.

Preferably, the arrangement paths within the ventricular myocardium extend along a long axis of a heart.

In this application, the pulse generator can be implanted into the body for generating electrical stimulation. The pulse generator itself is an existing technology, and the electrodes are electrically connected to the pulse generator through separate or integrated arrangements.

Preferably, the electrode and the pulse generator are formed separately, and wireless communication is used between the electrode and the pulse generator.

Preferably, the electrode and the pulse generator are formed separately, and the electrode has a smooth outer surface; or
the electrode and the pulse generator are formed integrally and have a smooth outer surface.

An outer surface of the above-mentioned electrode can be understood as the distal end and peripheral surface of the electrode, and the proximal end of the electrode can also be connected to a conducting wire.

The above-mentioned separate arrangement refers to the pulse generator being connected to theelectrodes through wires or wireless means. The pulse generator is generally implanted subcutaneously for easy portability, and can be used in existing conventional implantation sites or adjusted adaptively according to the direction of the electrode wires.

The above-mentioned integral arrangement refers to the integration of electrodes and pulse generators into a closed, capsule like integrated electrode.

When the electrodes are arranged separately, they all have smooth outer surfaces for easy puncture implantation into the ventricular myocardium. In addition, based on the structural characteristics of ventricular myocardium, smooth electrodes can be fixed without the need for existing structures such as spiral fixation.

Preferably, when the electrode and pulse generator are formed integrally, a capsule shaped integrated electrode is formed, which also integrates a battery unit for storing and stably releasing electrical energy, and a control unit for receiving, processing, and feedback signals. The integrated electrode has a smooth and closed shell.

Preferably, the pulse generator is located within the ventricular myocardium.

Most existing pulse generators are located subcutaneously, and with the development of miniaturization, in a preferred solution, they can also move along the implanted pathway of the electrode into the ventricular myocardium.

Preferably, the pulse generator has the same implantation pathway as the electrode.

Preferably, the outer periphery of the pulse generator is wrapped with flexible cushioning material.

In order to alleviate the unnecessary friction between the pulse generator and the ventricular v caused by heartbeat movement, a buffer material can be placed around the pulse generator. The buffer material can absorb ventricular myocardium movement, reduce direct friction with the pulse generator, and further improve safety. The buffer material can be made of medically acceptable materials, wrapped before the pulse generator is implanted, or injected around the pulse generator after implantation.

Preferably, the pulse generator adopts an internal or external power supply. The internal power supply eliminates the need for a power line, further improving integration.

In this application, the sheath as a whole is a tubular structure, with the interior serving as a channel for delivering electrodes. The electrodes can be releasably placed inside the sheath body, and its distal end is polished to form a tip through oblique cutting or other methods, making it easy to puncture into the body. The sheath is made of metal material with certain elasticity and necessary strength, and the diameter can be set according to the puncture object and electrode size.

Preferably, the length of the sheath body is 12~20cm.

Due to the need to enter the ventricular myocardium from outside the body, the sheath body should have sufficient length, which is also compatible and synergistic with the improvement of the implantation pathway related to the electrodes in this application.

Preferably, a steering wire extends movably through the sheath body, a distal end of the steering wire is connected to an inner wall of the puncture tip, and a proximal end of the steering wire extends out of the sheath body and serves as a force application end for pulling the puncture tip to change its orientation.

After entering the body, especially the ventricular myocardium, the angle of the sheath body may need to be changed during the puncture process. Due to the slender and elastic nature of the sheath body, pulling the steering wire at the proximal end can change the orientation of the puncture tip. The distal end of the steering wire can be fixed to the inner wall of the puncture tip by welding or connectors. The steering wire is made of a thin and strong metal wire to avoid spatial interference with electrodes and conducting wires in the sheath body as much as possible.

Preferably, further including a fixed handle with an internal transitional lumen. The proximal end of the sheath body is inserted into the fixed handle in a sealed manner, and an inner lumen of the sheath body communicates with the transitional lumen. The fixed handle is equipped with an electrode input port communicating with the transitional chamber, and the proximal end of the steering wire extends out of the fixed handle.

By means of setting the transitional lumen can separate the conducting wire from the steering wire at the proximal end, avoiding mutual interference during operation.

Preferably, a branch tube communicating with the transitional lumen is provided on a side wall of the fixed handle, and the steering wire extends out of the fixed handle from the branch tube.

The steering wire extends out of the branch tube on the side wall of the fixed handle, making the electrode and the conducting wire to be easily pushed along the axial direction of the sheath body.

Preferably, the cardiac rhythm adjustment system further includes a guiding device, which includes a base that can be fixed on the body surface and a cooperating seat that can be installed on the base with adjustable position. The sheath is assembled on the cooperating seat.

In this application, the sheath can be adjusted with the help of the guiding device, which can be in the form of a three-dimensional movable robotic arm, an adjusting bracket, etc. The sheath in different positions corresponds to different postures of the guiding device, which can be automatically or manually adjusted and controlled.

The guiding device of the present application includes a base that can be fixed on the body surface and a cooperating seat that can be installed on the base with adjustable position, and the sheath is assembled on the cooperating seat. The guiding device is used to locate the sheath and maintain various working states, wherein the working state refers to the puncture state in which the sheath moves relative to the guiding device.

Preferably, the guiding device is also used to maintain the sheath in various working states within a hemispherical space during the puncture process.

When the guiding device remains stationary as a whole, the sheath can move relative to the guiding device in a certain plane, and an angle between two extreme positions during the movement is 180 degrees.

If considering the movement of the guiding device itself or at least the movement of the portion carrying the sheath, the sheath can move within the hemisphere space.

Preferably, an arc-shaped guide rail is fixed on the base, the cooperating seat is installed on the arc-shaped guide rail in a slidable and positioning manner, and the sheath extends through the cooperating seat in a movable and positioning manner.

Preferably, the arc-shaped guide rail determines a first plane, and the steering wire pulls the sheath to bend in a distal second plane, wherein the first plane is perpendicular to the second plane.

Preferably, a fixed handle is provided at an end of the sheath away from the puncture tip, and the sheath cooperates with a guiding device through the fixed handle.

Preferably, the sheath body is configured with a scale indicating the relative position of the guiding device.

Preferably, further including an imaging system used to guide the sheath to puncture and display a position of the electrode.

Preferably, an ultrasound probe is provided on the cooperating seat and sends the position of the electrode and a posture of the sheath in real-time to the imaging system. The imaging system is at least one of ultrasound, CT, magnetic resonance imaging system, which is an existing technology, used to monitor the puncture position during surgery and display the position of the sheath and electrodes. The imaging system can collect, process data and display images. The sensing elements set inside and outside the patient's body to collect corresponding position state parameters can be used as detection units or connected to imaging equipment. When outputting images through the display unit, the information collected by the sensing elements can be added, which is more instructive.

Preferably, there is a navigation simulation system that provides indication relative positions of the electrical stimulation position and the at least one electrode in real-time. The navigation simulation system can be one or several of magnetic navigation system, infrared navigation system, and optical navigation system.

Preferably, the navigation simulation system performs a three-dimensional modeling of the heart in advance, and in use, the navigation simulation system collects electrode positions in real-time through an imaging system and displays them at corresponding positions of the heart model for comparison with the predetermined electrical stimulation phase. By collecting basic data of the patient's heart, including four items of heart damage data (TropI, Mb, CK MBmass, B-type natriuretic peptide), heart rhythm, routine and 24-hour dynamic electrocardiogram, ultrasonic electrocardiogram, ventricular wall thickness, location and degree of myocardial fibers, coronary artery shape, etc., a three-dimensional reconstruction of the patient's heart model can be obtained. Furthermore, the needle position can be collected and displayed in real-time in the heart model for real-time monitoring and guidance. The collection of basic data can be based on one or multiple of 3D ultrasound, CT, or MRI, followed by 3D modeling of the heart to display the structure of the heart, especially the interventricular septum, ventricular free wall, endocardium, and other related parts. By integrating ex vivo experiments, animal experiments, and clinical record data, the electrical stimulation position can be calculated and the corresponding implantation pathway can be planned.

Preferably, a pushing device is slidably set inside the sheath body, and a distal end of the pushing device is capable of pushing the electrode outside the sheath body for releasing.

During releasing the electrode, adequate thrust must be applied to ensure full implantation into the ventricular myocardium. Therefore, the cardiac rhythm adjustment system of this application introduces a pushing device to facilitate pushing the electrode into the ventricular myocardium.

Preferably, the pushing device is tubular-shaped and has an internal threading channel. The electrode is connected to a connecting wire, which extends towards the proximal end through the threading channel.

In this application, when faced with different scenarios of implanting one or more electrodes, the internal structure of the sheath body of the sheath is also improved accordingly to better adapt to different scenarios.

For example, the interior of the sheath can have a single or multiple lumens, with each lumen capable of independently arranging electrodes or serving as an extension channel for other components such as electrode wires, traction wires, and pushing devices.

Preferably, the sheath body includes a single lumen, and there are one or multiple electrodes arranged in sequence along the length direction of the sheath body.

The sheath body with multiple lumens can be equipped with various separating structures, such as setting a separating portion inside the sheath body to divide the sheath body into two lumens, or setting multiple independent auxiliary lumens inside the sheath body.

Preferably, an internal of the sheath body includes a first lumen and a second lumen that extend in parallel from a distal end to a proximal end, and the electrode includes multiple electrodes arranged along a length direction inside the first lumen; a separating portion is provided between the first lumen and the second lumen, and multiple avoidance holes are defined in the separating portion; at least one of the avoidance holes is provided at a proximal end side of each electrode, a connecting wire of each electrode extend from a corresponding avoidance hole to the second lumen and then out of the proximal end of the sheath body.

Preferably, damping structures acting with each other are provided between the first lumen and each electrode to avoid electrode displacement during the puncture process. The damping structure may be a protrusion on the inner wall of the sheath body that can hold the electrode, or damping generated between the sheath body and the electrode through interference fitting. Similar structures will not be listed one by one.

Preferably, a tubular-shaped pushing device is set inside the sheath body for receiving the connecting wire.

Preferably, pushing device can extend along the connecting wire through the corresponding avoidance hole into the first lumen to push the corresponding electrode.

The internal of the pushing device is a threading channel, and the connecting wire connected to the proximal end of the electrode extends towards the proximal end through the threading channel.

Preferably, multiple lumens are arranged side by side inside the sheath body, and each electrode is configured in a corresponding lumen;
a pushing device is slidably set inside the sheath body, and a distal end of the pushing device is capable of pushing the electrode outside the sheath body for releasing.

Preferably, there is/are one pushing device that can be switched between the lumens, or multiple pushing devices arranged in corresponding lumens.

Preferably, multiple electrodes are arranged in a staggered manner along the length direction of the sheath body.

In a preferred solution, the pushing device can be tubular-shaped with an internal threading channel. When the pushing device switches to one of the lumens, the connecting wire of the corresponding electrode in this lumen extends towards the proximal end through the threading channel.

In a preferred solution of multiple lumens, the electrode and the pulse generator can be arranged separately, and the electrode has a smooth outer surface; or, the electrode and the pulse generator are arranged in an integrated manner (i.e. forming an integrated electrode), and the integrated electrode has a smooth outer surface.

Preferably, the integrated electrode or the electrode has opposite proximal and distal ends, as well as outer peripheral surfaces extending between the distal and proximal ends. The distal and outer peripheral surfaces are smooth, and the proximal end is provided with a first junction position connected to the connecting wire in a removable manner.

Preferably, the first junction position can be a threading hole for extending of the connecting wire therethrough.

The electrode or integrated electrode mentioned above is connected to the connecting wire in a removable manner, which can be understood as having a threading hole on the electrode. When the connecting wire passes through the threading hole, the electrode is connected to the pushing device. When the electrode is implanted in place, the connecting wire can be removed from the electrode by cutting the connecting wire.

The present application also provides a heart rhythm adjustment method by implanting an electrode into a predetermined electrical stimulation position in the ventricular myocardium to perform corresponding electrical stimulation.

Preferably, during implanting electrodes, the sheath is first used to sequentially puncture the body surface and the epicardium to reach the electrical stimulation position inside the ventricular myocardium, and then the electrode is delivered to the electrical stimulation position through the lumen of the sheath body of the sheath.

Preferably, implanting the electrode to the predetermined electrical stimulation position in the ventricular myocardium includes:
planning an implantation pathway;
providing a sheath with pre-installed electrode, puncturing the sheath along the implantation pathway through the epicardium until a distal end of the sheath reach the predetermined electrical stimulation position; and releasing the electrode from the sheath and maintaining it at the predetermined electrical stimulation position under the coverage of surrounding tissues.

Preferably, planning the implantation pathway includes:
collecting basic data of the heart;
performing 3D-modeling of the heart to obtain a heart model, wherein collecting the basic data is based on one or multiple of three-dimensional ultrasound, CT, magnetic resonance imaging; and structures of an interventricular septum, a ventricular free wall, and an endocardium are displayed in the heart model; and
calculating the electrical stimulation position and planning implantation pathway correspondingly.

Preferably, collecting the position of the sheath in real-time during epicardial puncture along the implantation pathway, and displaying the position of the sheath in the heart model in real-time through calculation and simulation. Preferably, a connecting wire is pre-connected to the proximal end of the electrode in a removable manner.

Preferably, when the electrode is detached from the sheath, it is pushed towards the distal end along the connecting wire of the electrode until the electrode is pushed out of the sheath body.

Preferably, the sheath is equipped with a slidable pushing device, and the distal end of the pushing device can push the electrode to release it from the sheath body.

Preferably, a first lumen and a second lumen are formed inside the sheath through a separating portion. A pushing device with a threading channel is slidably arranged inside the second lumen. Multiple electrodes are arranged along the length direction inside the first lumen, and the separating portion is provided with multiple avoidance holes. The proximal end of each electrode is connected with a connecting wire, which extends from a corresponding avoidance hole through the threading channel and out of the proximal end of the sheath body. Removing the electrode from the sheath includes:
S100, when the distal end of the sheath reaches the farthest electrical stimulation position, pushing the pushing device towards the distal end along the connecting wire of the farthest electrode, making the pushing device enter the first lumen through corresponding avoidance hole; and pushing the pushing device towards the distal end continuously until the farthest electrode is pushed out of the sheath;
S200: retracting the sheath to the secondary farthest electrical stimulation position, and then pushing the pushing device towards the distal end along the connecting wire of the secondary farthest electrode, making the pushing device enter the first lumen through corresponding avoidance hole; and pushing the pushing device towards the distal end continuously until the electrode at the secondary distal end is pushed out of the sheath; and
repeating the above steps until all of the electrodes are pushed out of the sheath.

In this application, the electrodes are implanted via an intramyocardial pathway and controlled by a pulse generator program to achieve real physiological position pacing and biventricular electrical synchronization. Although the mechanisms of malignant ventricular arrhythmia vary, the substrate that causes malignant ventricular arrhythmia is generally located in a fixed position that can be easily reached by the pacing electrodes within the myocardium. In this way, local rapid pacing can suppress or low-energy defibrillation, achieving the goal of suppressing malignant ventricular arrhythmias.

### BRIEF DESCRIPTION OF THE DRAWINGS

Fig. 1 is a schematic view showing an implantation site of an electrode according to an embodiment of the present application.
Fig. 1a is a schematic view showing a puncture implantation process of the electrode of Fig. 1.
Fig. 2 is a schematic view showing an implantation site of an electrode of an embodiment.
Fig. 3 is a schematic view showing an implantation site of an electrode of an embodiment.
Fig. 4 is a schematic view showing an implantation site of an electrode of an embodiment.
Figs. 4a-4d are schematic views showing a puncture implantation process of the electrode of Fig. 4.
Fig. 4e is a schematic view showing a puncture implantation process of the electrode of an embodiment.
Fig. 4f is a schematic view of an electrode of an embodiment.
Fig. 5 is a schematic view showing an implantation site of an electrode of an embodiment.
Fig. 6 is a schematic view showing the implantation site of the electrode of Fig. 5 from another aspect.
Fig. 7 is a schematic view showing an implantation site of an electrode of an embodiment.
Fig. 8 is a schematic view showing an implantation site of an electrode of an embodiment.
Fig. 9 is a schematic view showing an implantation site of a reference electrode of an embodiment.
Fig. 10 is a schematic view showing an implantation site of a pulse generator of an embodiment.
Fig. 11 is a schematic view of a sheath.
Fig. 12 is a schematic view of a fixed handle of the sheath.
Fig. 13 is a schematic view of a connection part of a steering wire of the sheath.
Fig. 14 is a schematic view showing the change in position of the sheath.
Fig. 15 is a schematic view of an imaging system.
Fig. 16 is a schematic view of a guiding device.
Figs. 17-19 are schematic views showing different connection manners between the electrode and the pulse generator of the present application.
Figs. 20-21 are schematic views showing different electrodes of the present application.
Fig. 22 is a schematic view showing a pathway for inserting the electrode via the lower limb veins of an embodiment of the present application.
Fig. 23 is a schematic view of an electrode through the systemic vein to the right ventricle of one embodiment of the present application.

Reference numbers in the accompanying drawings are as follows:
1, left ventricle; 11, right atrium; 2, right ventricle; 3, interventricular septum; 4, cardiac apex; 5, epicardium; 6, endocardium; 7, ventricular free wall; 8, His bundle; 9, right bundle branch; 10, left bundle branch; 12, sheath body; 121, first lumen; 122, second lumen; 123, separating portion; 124/124a, avoidance hole; 13, puncture tip; 14, fixed handle; 16, sealing head; 17, branch tube; 18, transitional lumen; 19, steering wire; 20, sheath; 21, conducting wire; 30, guiding device; 31, cooperating seat; 32, base; 33, ultrasonic probe; 40, imaging system; 50, pushing device; 51, pushing tube; 52, threading channel; 53, threading hole; 54, connecting wire; 60/61/62/63/64/65/66, electrode; 601, anchor; 602, support body; 70, reference electrode; 80, pulse generator; 90, body surface.

### DESCRIPTION OF THE EMBODIMENTS

The following will provide a clear and complete description of the technical solutions in the embodiments of the present application, in combination with the accompanying drawings. Obviously, the described embodiments are only a part of the embodiments of the present application, not all of them. Based on the embodiments of this application, all othesr embodiments obtained by those skilled in the art without creative labor are within the scope of protection of this application.

It should be noted that when a component is referred to as "connected" to another component, it may be connected to another component directly or there may be a middle component connected therebetween. When a component is considered to be "set on" another component, it may be set on another component directly or there may be a middle component set therebetween.

Unless otherwise defined, all technical and scientific terms used in this disclosure have the same meanings as those commonly understood by those skilled in the art belonging to the present application. The terms used in the disclosure of this application are only for the purpose of describing specific embodiments and are not intended to limit the scope of this application. The term "and/or" used in this disclosure includes any and all combinations of one or more related listed items.

An embodiment of the present application provides a cardiac rhythm adjustment system and a cardiac rhythm adjustment method, specifically by implanting an electrode via the epicardium, which may also be understood as providing a method of implanting electrode via the epicardium. The relevant components of the cardiac rhythm adjustment system may also be combined with the relevant description of the implant delivery system (delivery system) in the following text.

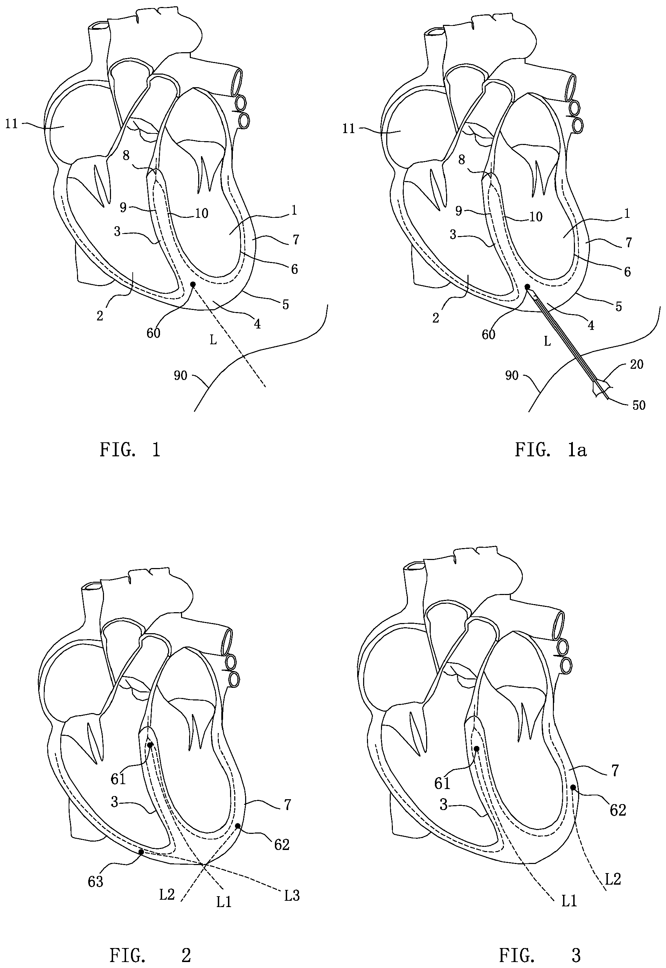

Referring to Fig. 1, an internal structure of a heart is shown, wherein an interventricular septum 3 is located between a left ventricle 1 and a right ventricle 2, a right atrium 11 is above the right ventricle 2, and an epicardium 5 and an endocardium 6 are respectively provided at an internal and an external of a ventricular myocardium. In Fig. 1, a cardiac apex 4 is located at a bottom of the heart. The ventricular myocardium includes the interventricular septum 3 (interventricular septum myocardium) and a ventricular free wall 7 (free wall myocardium). Conduction bundle (indicated by dashed lines) extends inside the ventricular myocardium, specifically including His bundle 8. A distal end of the His bundle 8 branches into two branches, i.e., a left bundle branch 10 and a right bundle branch 9.

In order to regulate heart rhythm, an electrode 60 is implanted into the interventricular septum 3 at a position slightly close to the cardiac apex 4. An implantation pathway L of the electrode 60 extends into the ventricular myocardium from a body surface 90 via the cardiac apex 4. In case that wired connection is used for the electrode, a portion of the implantation pathway L within the ventricular myocardium may also be considered as an extending direction of a conducting wire. An internal structure of the electrode 60 has been described in relevant prior arts.

In this embodiment, one electrode 60 is implanted. The electrode 60 is located not only at the position shown in Fig. 1, i.e., within the interventricular septum 3 and away from the His bundle 8, but also in other embodiments within the interventricular septum 3 and adjacent to the end branches of the His bundle 8 or within the ventricular free wall 7 but at least within the ventricular myocardium between the epicardium 5 and the endocardium 6. The electrode 60 located within the interventricular septum myocardium may be a pacemaker electrode. The electrode 60 located within the free wall pacemaker of the ventricle is a pacemaker electrode and/or a defibrillation electrode.

During the implantation process, the operator implants the electrode 60 into a predetermined electrical stimulation position through the cooperation of a guiding device, a sheath, a pushing device, and an imaging system.

This embodiment also provides a method for implanting the electrode, including:
S100: puncturing through the epicardium to deliver a delivery system along an implantation pathway (in other embodiments described below, other interventional pathways for implanting the electrode 60 are also provided) to a predetermined first implantation site (electrical stimulation position);
S101: releasing a first implanted electrode to the first implantation site; and
S102: withdrawing the delivery system.

In this embodiment, the implantation method further includes: before step S100, providing a sheath with a pre-installed electrode. The electrode is pre-installed into the sheath, and a connecting wire connected to the electrode extends through the sheath body.

The above implantation method further includes: before step S100, planning an implantation pathway through an imaging system, which specifically includes:
collecting basic data of the heart, such as chamber size, long and short axis of the heart, and etc.;
perform 3D-modeling of the heart to obtain a heart model, wherein the connection of basic data is based on one or multiple of three-dimensional ultrasound, CT, magnetic resonance imaging; and the structures of the interventricular septum, ventricular free wall, and endocardium are displayed in the heart model; and
calculating an electrical stimulation position and planning a corresponding implantation pathway.

As shown in Fig. 1a, along a direction of the planned implantation pathway, the sheath pericardial puncture approach: the puncture pathway of the sheath is positioned on the planned implantation pathway L, and the sheath can be maintained in a working state of extending along the implantation pathway L to puncture. When the sheath extends along the implantation pathway and pericardial puncture approach, a real-time position of the sheath can be collected by an ultrasound probe, and the position of the sheath in the heart model can be displayed in real-time through calculation and simulation.

When the implanted electrode and the pulse generator are formed separately and communicate with each other through wired connection, the above implantation method further includes: after step 102, connecting the conducting wire connected to the electrode to the pulse generator and implanting the pulse generator into subcutaneous tissues of the human body.

When the implanted electrode and the pulse generator are formed separately and communicate with each other through wireless communication, the above implantation method further includes: after step 102, implanting the pulse generator into subcutaneous tissues of the human body.

When the implanted electrode and the pulse generator are formed integrally, in step S101, a removable connecting wire may be pre-connected to a proximal end of the electrode, so that when the electrode is detached from the sheath, the electrode can be pushed along the connecting wire towards the distal end until it is pushed out of the sheath body.

The above implantation method further includes: after step S101, retracting the delivery system to a second implantation site and releasing a second implanted electrode at the second implantation site. After implanting the second electrode, a third electrode may be released at a third implantation site according to actual case.

In an embodiment, one electrode is pre-installed in the delivery system. When completion the implantation of this electrode, a second electrode is loaded into a pushing tube. A specific implementation method is as follows:
The electrode 60 is disposed in the sheath body of the sheath in a releasable manner, and a damping structure, such as a protrusion for restricting a sliding of the electrode 60, may be set on an inner wall of the sheath body.

The pushing device is a hollow pushing tube configured with a certain strength. A distal end of the pushing tube may also be connected to the electrode 60 in a releasable manner. For example, the distal end of the pushing tube is fixed with a connecting wire, and a proximal end of the connecting wire extends out of a proximal end of the pushing tube after passing through a joint of the electrode.

Specific operations include:
1). connecting the electrode 60 to a distal end of the pushing tube 51 via a connecting wire in a releasable manner, wherein the electrode 60 is set inside the sheath body, and the proximal end of the pushing tube 51 extends out of the proximal end of the sheath body;
2). making the puncture tip 13 of the sheath 20 directly move to the electrical stimulation position along the implantation pathway L through the guiding device 30;
3). pushing the electrode 60 to a predetermined electrical stimulation position through the pushing tube 51;
4). determining whether the releasing position meets expectations through the radiography of the imaging system; and
5). removing the connecting wire while maintaining the position between the electrode 60 and the pushing tube 51, and retracting the sheath 20 and the pushing tube 51 simultaneously outside the body after the connecting wire is completely removed.

A detailed description of the implantation sites for the electrodes is as follows.

Referring to Fig. 2, in this embodiment, at least two electrodes are implanted into the myocardium to regulate heart rhythm. The electrode 61 is implanted into the interventricular septum 3 at a position near the end branches of the His bundle 8, and the implantation pathway L1 of the electrode 61 extends to the interventricular septum 3 from the body surface via the cardiac apex 4.

In addition, two electrodes 62, 63, corresponding to implantation pathways L2, L3, respectively, are implanted in the ventricular free wall 7. The implantation pathways L2, L3 extend respectively to the ventricular free wall 7 at a corresponding side from the body surface via the cardiac apex 4, wherein the implantation pathways L1, L2, and L3 extend to the myocardium via the same puncture point.

In case of wired connection, a portion of each implant pathway within the ventricular myocardium may also be considered as an extending direction of the conducting wire.

The portion of each implantation pathway within the ventricular myocardium is located between the epicardium 5 and the endocardium 6, and efforts are made to avoid puncturing the conduction bundle. When switching between different implantation pathways during implantation, the sheath may always remain within the cardiac apex and directly change direction to switch implantation pathways after retracting to the vicinity of the cardiac apex.

As an embodiment of the present application, different implantation pathways may also enter the myocardium through different puncture points. Referring to Fig. 3, in this embodiment, in order to regulate heart rhythm, an electrode 61 is implanted into the interventricular septum 3 at a position near the end branches of the His bundle 8. The implantation pathway L1 of the electrode 61 extends to the interventricular septum 3 from the body surface via the cardiac apex 4.

In addition, an electrode 62, corresponding to implantation pathway L2, is implanted into the ventricular free wall 7. The implantation pathway L2 extends to the ventricular free wall 7 from the body surface via the epicardium at the ventricular free wall 7.

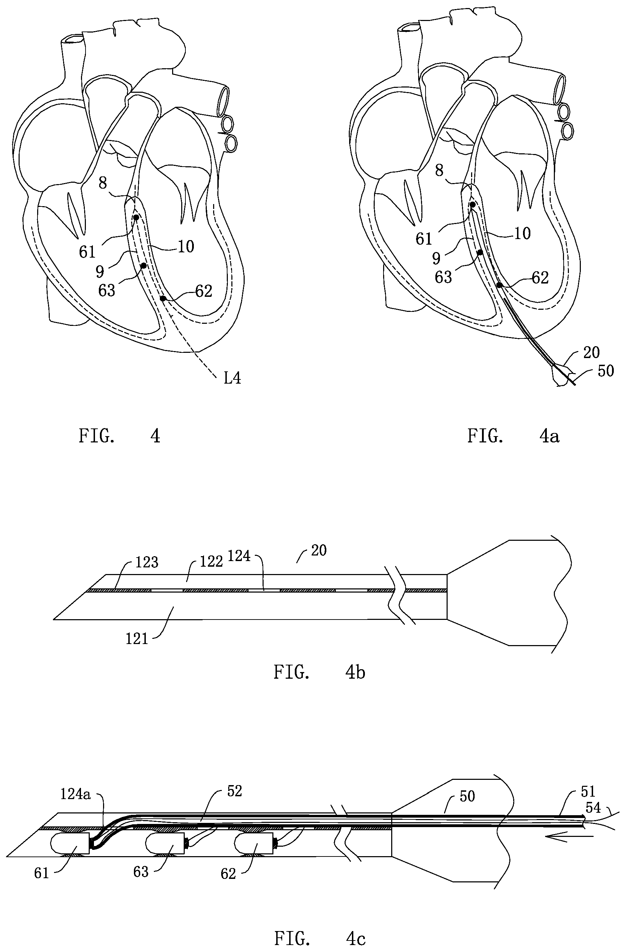

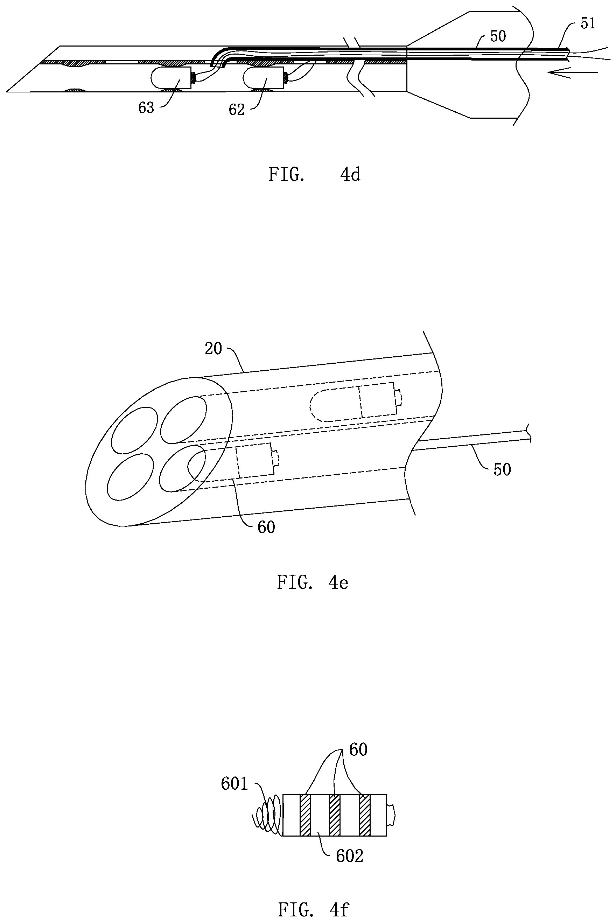

As an embodiment of the present application, multiple electrodes may be implanted onto the same implantation pathway. Referring to Fig. 4, in this embodiment, an electrode 61 is implanted into the interventricular septum 3 at a position near the distal branches of the His bundle 8 to regulate heart rhythm; an electrode 63 is implanted into a middle position of a long axis of the interventricular septum 3 and near the right bundle branch 9; an electrode 62 is implanted into the interventricular septum 3 at a position adjacent to the cardiac apex and the left bundle branch 10. In this embodiment, the multiple electrodes are generally arranged in a straight line and can be implanted through the same implantation pathway L4, such as entering the interventricular septum 3 from the body surface via the cardiac apex 4.

Referring to Figs. 3-4 and 4a-4d, when the sheath needs to implant multiple electrodes along one or multiple implantation pathways, an interior of the sheath body 12 includes a first lumen 121 and a second lumen 122 which are arranged side by side and extend from the distal end to the proximal end. A separating portion 123 is provided between the first lumen 121 and the second lumen 122. Multiple electrodes are arranged along a length direction inside the first lumen.

There are multiple avoidance holes 124 defined in the separating portion 123. For each electrode, at least one avoidance hole is provided at a proximal end side of the electrode. Each electrode is connected to a detachable connecting wire 54, and the connecting wires extend respectively through the avoidance holes to the second lumen and then out of the proximal end of the sheath body.

When the puncture tip of the sheath reaches the electrical stimulation position directly, a pushing device 50 is set inside the first lumen to push the electrode inside the second lumen. The pushing device 50 is a hollow tubular structure, such as the pushing tube 51 shown in the figure. An internal of the pushing tube is a threading channel 52 for the connecting wire. The pushing device can move along the connecting wire to enter the first lumen through corresponding avoidance hole to push corresponding electrode.

Specifically, the implantation process of multiple electrodes will be described as follows.

In another embodiment, the multiple electrodes are pre-installed inside the delivery system simultaneously.

Referring to Figs. 4a to 4d, the guiding device 30 locates the puncture point and puncture angle of the sheath. The multiple electrodes, namely electrode 61, electrode 63, and electrode 62, are arranged along the length direction in the first lumen 121 of the sheath. These electrodes correspond to predetermined implantation sites in a one-to-one order. A damping structure is provided between the first lumen and each electrode to prevent electrode displacement during the puncture process.

Each electrode has a pre-connected removable connecting wire 54, which extends from the avoidance hole closest to the proximal end of the corresponding electrode into the second lumen and then out of the proximal end of the sheath body. As shown in Figs. 4c and 4d, the connecting wire of the electrode 61 extends from the avoidance hole 124a to the second lumen, and the same applies to other electrodes.

The pushing device 50 is set in the second lumen, and can at least move along the connecting wire through the corresponding avoidance hole to push the corresponding electrode. The pushing device is a pushing tube that cooperates with the connecting wire in a slidable manner, and the distal end of the pushing tube pushes the electrode.

More specifically, the implantation process of multiple electrodes is as follows:
S100: puncturing through the epicardium, making the delivery system reach a predetermined implantation site (the sheath reaches the farthest electrical stimulation position); pushing the pushing device towards the distal end along the connecting wire of the farthest electrode, making the pushing device enter the first lumen through corresponding avoidance hole; and pushing the pushing device towards the distal end continuously until the farthest electrode is pushed out of the sheath;
S200: retracting the sheath to the secondary farthest electrical stimulation position, and then pushing the pushing device towards the distal end along the connecting wire of the secondary farthest electrode, making the pushing device enter the first lumen through corresponding avoidance hole; and pushing the pushing device towards the distal end continuously until the electrode at the secondary distal end is pushed out of the sheath;
repeating the above steps until all of the electrodes are pushed out of the sheath. Finally, the sheath and the pushing device are retracted, wherein the operation may refer to the previous embodiments.

In another embodiment, as shown in Fig. 4e, during the implantation process of multiple electrodes, the sheath body may include multiple lumens arranged in parallel, and each electrode is configured in one corresponding lumen. The pushing device is slidably set inside the sheath body, and the distal end of the pushing device can push the electrode to release it out of the sheath body. There may be one pushing device capable of switching between different lumens, or, there are multiple pushing devices each arranged in one corresponding lumen. The multiple electrodes may be arranged in a staggered manner along the length direction of the sheath body.

In another embodiment, as shown in Fig. 4f, the multiple electrodes 60 may be set on the same support body 602. The support body 602 may be made of an acceptable biocompatible material to keep the multiple electrodes 60 insulated from each other. The electrode 60 is annular-shaped and fixed around an outer circumference of the support body 602. There may be 2~3 electrodes 60 arranged at intervals, which can be implanted together with the support body 602. An end of the support body 602 may be equipped with an anchor 601 that is spiral wound and converge in shape, used to screw into surrounding tissues to maintain it in a predetermined position. The anchor 601 may be made of metal material, and it can also be connected to a circuit and used as an electrode.

Of course, in other embodiments, one, two, or more electrodes may be implanted based on the patient's symptoms. The multiple electrodes may be implanted along multiple implantation pathways through the same puncture point, or along multiple implantation pathways through different puncture points.

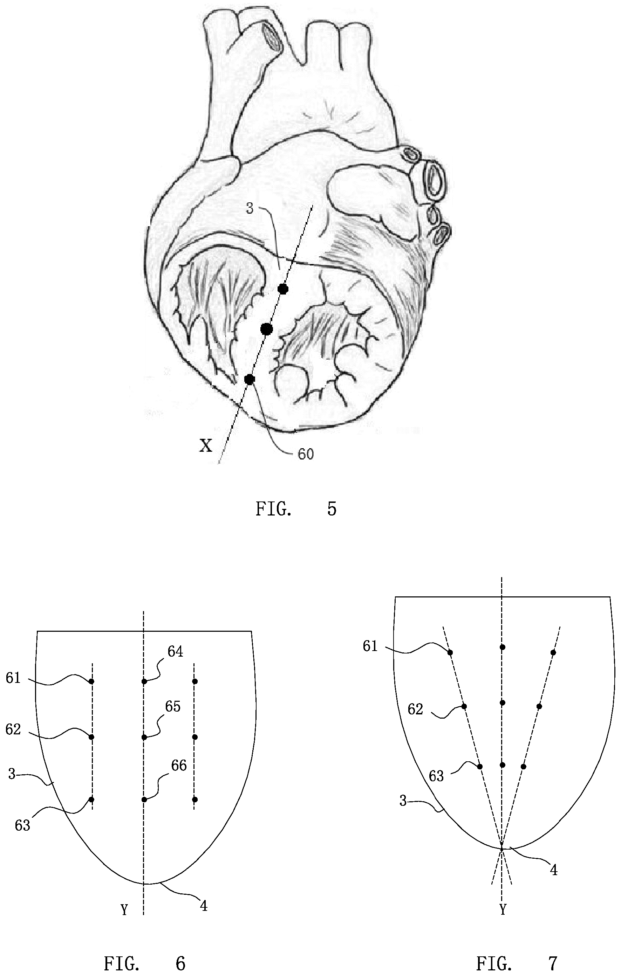

Referring to Figs. 5 and 6, in this embodiment, multiple electrodes A are implanted into the interventricular septum 3 to regulate heart rhythm. The electrodes are divided into three groups which are arranged along a short axis X of the interventricular septum 3. The three electrodes belonging to the same group are arranged along the long axis Y of the interventricular septum 3.

For example, the electrodes 61, 62, and 63 are configured as one group and arranged along the long axis Y of the interventricular septum 3. The three electrodes 64, 65, and 66 in an adjacent group are also arranged along the long axis Y of the interventricular septum 3.

All of the three groups of electrodes can be implanted through apex puncture. In this embodiment, the electrodes of each group are generally arranged in a straight line. Of course, in other embodiments, the number of groups and the number of electrodes in each group may be adjusted according to the needs and characteristics of the lesion site. For example, there may be two or four groups of electrodes, and each group has two or four electrodes. The number of electrodes in the groups may be the same or different. For example, in this embodiment, the number of electrodes in the three groups may be one, two, and three, respectively; or, the number of electrodes in the three groups may be two, two, three, and etc.

As an embodiment of the present application, referring to Fig. 7, compared to the embodiment shown in Fig. 5, in this embodiment, the electrodes of each group in the interventricular septum 3 are generally arranged in a straight line, and the three groups of electrodes are generally distributed radially around the cardiac apex 4, that is, entering the interventricular septum along the same puncture point.

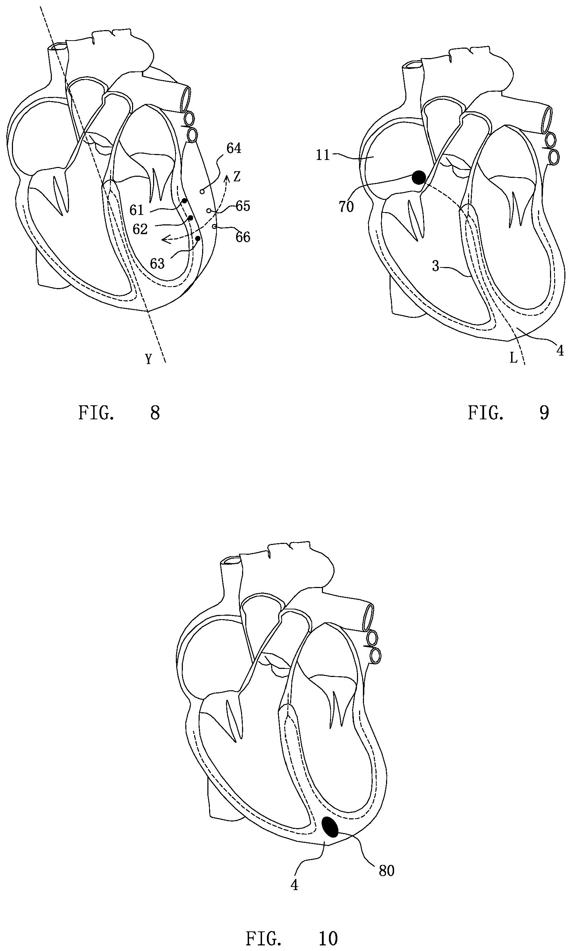

The middle group of electrodes is arranged along the long axis Y of the interventricular septum 3, while the other groups on two sides of the middle group are arranged at an angle relative to the long axis Y of the interventricular septum 3. For example, the electrodes 61, 62, and 63 of the left group has a certain angle, such as 15 to 60 degrees, with the long axis Y of the interventricular septum 3. As an embodiment of the present application, referring to Fig. 8, in this embodiment, multiple electrodes are implanted into the ventricular free wall 7 to regulate heart rhythm. This embodiment may also be combined with other embodiments, that is, on the basis of implanting electrodes into the interventricular septum, electrodes are further implanted in the ventricular free wall 7. Of course, electrodes may also be implanted only in the ventricular free wall 7.

The multiple electrodes are divided into two groups, all located between the epicardium 5 and the endocardium 6. For example, the electrodes 61, 62, and 63 are arranged as a group along the long axis Y of the interventricular septum 3. The three electrodes 64, 65, and 66 in an adjacent group are also arranged along the long axis Y of the interventricular septum 3.

The two groups of electrodes are distributed along the circumferential direction Z of the ventricular free wall 7. The implantation pathway of each group of electrodes may follow the implantation pathway of the electrodes in the ventricular free wall 7 of the embodiment shown in Fig. 2 or Fig. 3.

In other embodiments, reference electrodes may be increased as needed and implanted in corresponding positions.

Referring to Fig. 9, in this embodiment, in order to obtain electrocardiogram signals as a reference for regulating heart rhythm, a reference electrode 70 is implanted in the right atrium 11. The implantation method and arrangement of the electrode used for heart rhythm adjustment can be combined with other embodiments. In this embodiment, the implantation pathway L of the reference electrode 70 extends to the right atrium 11 from the body surface through the cardiac apex 4 and the interventricular septum 3 sequentially. The anchoring method of the reference electrode 70 and the electrode used for cardiac rhythm adjustment at their respective positions may refer to existing methods, such as active or passive methods.

Referring to Fig. 10, in order to drive the electrodes, corresponding pulse generator need to be matched. Most existing pulse generators are implanted subcutaneously. In this application, an embodiment of implanting the pulse generator 80 into the ventricular myocardium is also provided. The implantation pathway of the pulse generator 80 is from the body surface, through the cardiac apex 4 and into the interventricular septum. The position of the pulse generator 80 within the interventricular septum is preferably located at the cardiac apex where the conduction bundle is relatively sparse. In a preferred embodiment, an external of the pulse generator 80 is wrapped with a flexible cushioning material that can absorb movement of the ventricular myocardium, reducing direct friction with the pulse generator, and further improving safety. The cushioning material can be medically acceptable, being wrapped before the pulse generator is implanted or injected around the external of the pulse generator after implantation. The pulse generator 80 adopts an internal or external power supply, wherein the internal power supply can eliminate the need for a power line, further improving integration.

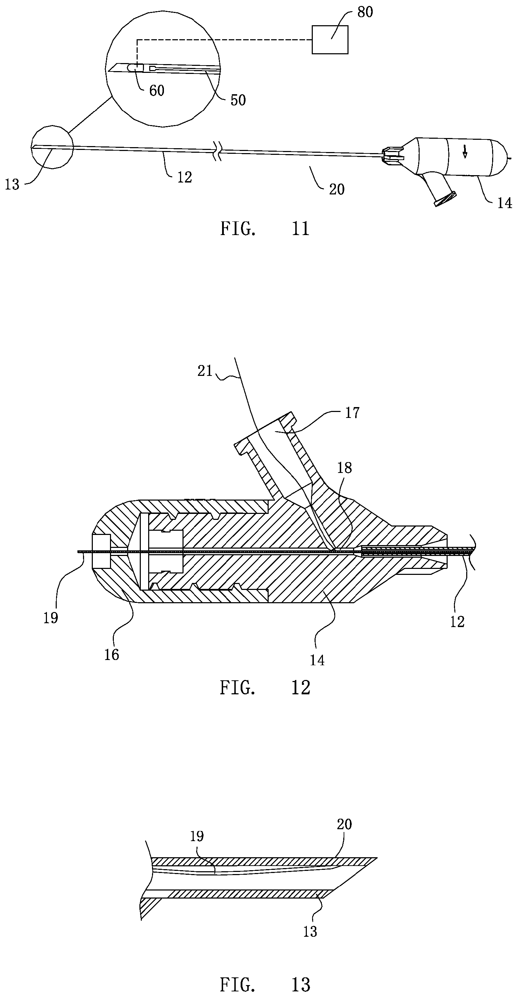

Referring to Figs. 11-13, for the convenience of implantation operation, the sheath includes a hollow sheath body 12. A distal end of the sheath body 12 is a puncture tip 13 that can penetrate into the ventricular myocardium and guide the sheath body 12 therethrough, and a proximal end of the sheath body 12 is a fixed handle 14.

The sheath is a tubular structure as a whole, with an interior serving as a channel for delivering the electrodes. The distal end of the sheath is formed into a tip through oblique cutting and other polishing methods, making it easy to puncture into the human body. The sheath is made of metal material with certain elasticity and necessary strength, and its diameter can be set according to the puncture object and electrode size.

The sheath body 12 has a length at least can extend from the outside of the body through the cardiac apex into the ventricular myocardium, for example, 12~20cm.

An internal of the fixed handle 14 is configured with a transitional lumen 18, and the sheath body 12 is connected to the transitional lumen 18. A side wall of the fixed handle 14 is provided with a branch pipe 17 that communicates with the transitional lumen 18. A sealing head 16 is screwed onto a proximal end of the fixed handle 14. A threading hole communicating with the transitional lumen 18 is defined in the sealing head 16. The connecting wire (i.e., the conducting wire), which is connected to the electrode, can extend out of the proximal end of the fixed handle 14 via the threading hole.

During implantation, in order to adjust the implantation pathway, the delivery system further includes a steering device, which may be configured as a steering wire. For example, as shown in Fig. 13, a steering wire 19 extends movably through the sheath body 12. The steering wire 19 is made of a thin and high-strength metal wire. A distal end of the steering wire 19 is welded and fixed to an inner wall of the puncture tip 13, and a proximal end of the steering wire 19 extends out of the fixed handle 14 and acts as a force application end for pulling the puncture tip 13 to change its orientation (as shown in Fig. 13). The proximal end of the steering wire 19 extends out of the side wall of the fixed handle 14 from the branch pipe 17. Alternatively, the connecting wire 21 connected to the electrode may extend out of the fixed handle 14 from the branch pipe 17, and the steering wire 19 may extend out of the proximal end of the fixed handle 14 from the threading hole (as shown in Fig. 12).

The conducting wire 21 and the steering wire 19 are routed separately to avoid mutual interference during operation, which facilitates the pushing of the electrodes and the connecting wires along the axial direction of the sheath body 12.

This application provides an implant delivery system that includes a sheath and an implant. The sheath includes a hollow sheath body, in which the implant can be set in a releasable manner. A distal end of the sheath body is a puncture tip that can penetrate the ventricular myocardium and guide the sheath body therethrough. A length of the sheath body can extend to the ventricular myocardium from the outside of the body via the cardiac apex. The implants may be hydrogels, therapeutic drugs, electrodes, and etc. The implant delivery system of this application is suitable for implantation through the intramyocardial pathway, reducing operational difficulty.

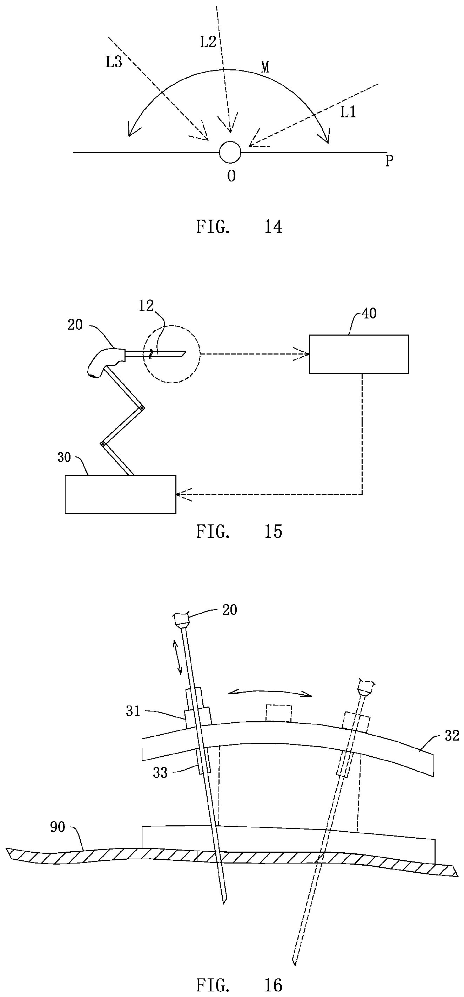

Referring to Figs. 14 to 16, in an embodiment, in order to achieve precise positioning during implantation, the implant delivery system further includes a guiding device 30, which is used to puncture position of the sheath 20 and maintain its working state. The guiding device 30, for example, may be configured as a robotic arm that can move in three-dimensional space, in the form of an adjustable bracket, etc. For example, the guiding device 30 includes a base 32 that can be fixed onto the body surface 90 and an cooperating seat 31 which is installed on the base 32 and adjustable in position. The sheath 20 is assembled to the cooperating seat 31, and the base 32 that can be fixed to the body surface 90 is tightly attached to the body surface 90 and fixed in position relative to the body surface 90. The base 32 may be rigid or a flexible restraint band.

The guiding device 30 is relatively fixed on the body surface 90 through the base 32, and the sheath 20 is assembled to the cooperating seat, so that the puncture point and puncture angle of the sheath 20 are positioned, that is, through the cooperation of the cooperating seat 31 and the base 32, the puncture pathway of the sheath 20 can be positioned on the planned implantation pathway L, and the sheath 20 can maintain the working state of extending along the implantation pathway L to puncture.

In an embodiment, an arc-shaped guide rail is fixed onto the base 32, and the cooperating seat 31 is installed and positioned on the arc-shaped guide rail in a slidable manner. The sheath 20 extends through the cooperating seat 31 for movable positioning. The cooperating seat 31 slides along the arc-shaped guide rail, wherein the arc-shaped line determines a first plane, the steering wire 19 pulls the sheath 20 to bend in a second plane at the distal end, wherein the first plane is perpendicular to the second plane.

In this embodiment, the fixed handle of the sheath 20 can be slidably installed on the cooperating seat 31, and the sheath body of the sheath 20 is also provided with a scale indicating the relative position of the guiding device 30.

Referring to Fig. 14, taking the height of the heart position O as a reference, a reference horizontal plane P can be assumed. Under the guidance of the guiding device 30, the sheath 20 can move in a hemispherical space above the reference horizontal plane P and maintain a predetermined angle.

When the cooperating seat 31 of the guiding device 30 remains stationary, the sheath 20 can only slide, for example, slide back and forth along the implantation pathway L1. If the relative position relationship between the sheath 20 and the cooperating seat 31 is maintained, and only the cooperating seat 31 is moved, for example, simplifying the two-dimensional movement of the sheath 20 in a certain plane, an angle M between two extreme positions of the sheath 20 during movement is 180 degrees. When the implantation pathway needs to be switched, driving the cooperating seat 31 can make the sheath 20 move along the implantation pathway L1, the implantation pathway L2, the implantation pathway L3, respectively.

The system of this application can also be equipped with an imaging system 40 for real-time display of the treatment site and sheath, which specifically may be at least one of an ultrasound imaging system, a CT imaging system, a magnetic resonance imaging system. The implant delivery system of this application may also be equipped with a navigation simulation system for real-time display of the treatment site and sheath.

The navigation simulation system first performs a three-dimensional modeling of the heart. During use, the navigation simulation system collects the position of the treatment needle in real-time through an imaging device and displays the corresponding position in the heart model. Preferably, the guiding device is equipped with an ultrasound probe 33, which sends real-time collected electrode positions or distal posture of the sheath to the imaging system. The navigation simulation system plans the implantation pathway and treatment position of electrical stimulation based on the heart model, and can control the guiding device to adjust the movement of the sheath.

In an embodiment of the present application, the system also includes a biopsy needle for puncturing ventricular myocardium for biopsy. The use of biopsy needles for tissue extraction before, during, and after surgery can reveal changes in the lesion site and evaluate surgical outcomes. Tissue extraction may include body fluids, muscles, and etc. The sheath may also serve as a biopsy needle. Taking implanting electrodes as an example, the structure of the delivery system will be described in detail.

The implant delivery system of the present application includes at least one electrode 60 that can be implanted into the ventricular myocardium, a pulse generator 80, and a sheath 20. The pulse generator 80 is used to generate electrical stimulation, and the electrode 60 is electrically connected to the pulse generator 80 by way of being formed separately and then connected together or formed integrally.

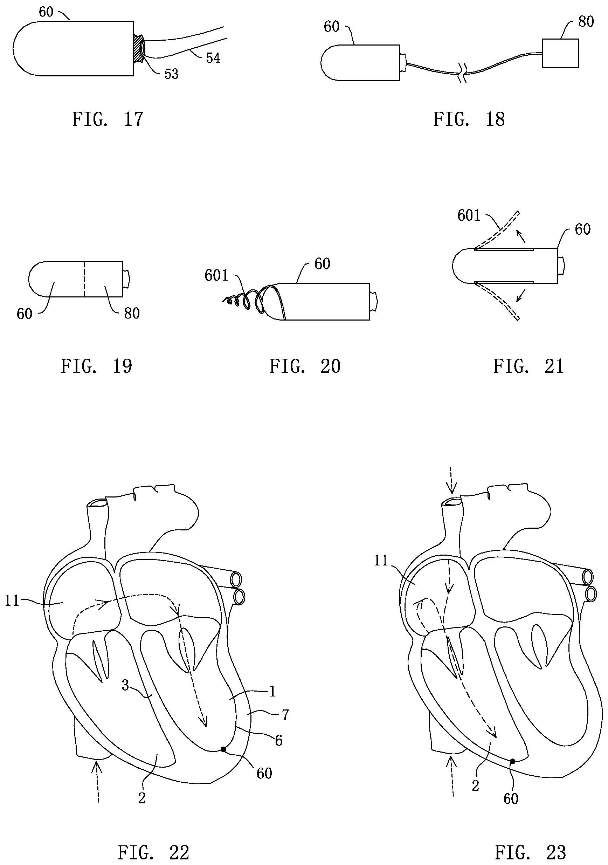

As shown in Fig. 17, the proximal end of the electrode 60 is connected to the connecting wire 54 through the threading hole 53. When the electrode 60 and the pulse generator 80 are integrated and packaged together, or when the electrode 60 is connected to the pulse generator 80 through wireless communication, the connecting wire 54 serves as a traction wire to connect the electrode 60 to the delivery system. When the electrode 60 is delivered to the implantation site, the electrode 60 is released by retracting the traction wire. When the electrode 60 is integrated and packaged together with pulse generator 80, the connecting wire 54 serves as a traction wire. When the electrode 60 is delivered to the implantation site, the electrode 60 is released by retracting the traction wire. When the electrode 60 is connected to the pulse generator 80 in a wired manner for communication, the connecting wire 54 serves as a conducting wire used to establish electrical connection between the electrode 60 and the pulse generator 80.

As shown in Fig. 18, for example, when the electrode 60 and the pulse generator 80 are formed separately and then connected together, the electrode 60 is electrically connected to pulse generator 80 through a conducting wire, or wireless connection is adopted to communicate with the pulse generator. During implantation, the pulse generator may be placed in a subcutaneous pouch next to the sternum using a common manner of implanting pacing electrode.

As shown in Fig. 19, when the electrode 60 and the pulse generator 80 are formed integrally, the electrode 60 and the pulse generator 80 are integrated and packaged together, for example, including a housing and a pulse generator 80 located inside the housing, wherein the electrode 60 is exposed outside the housing. As an embodiment, the housing is capsule shaped, and the electrode 60 extends out from one end of the housing. The sheath includes a hollow sheath body, and the electrodes can be placed inside the sheath in a releasable manner.

As shown in Fig. 20, in an embodiment, in order to anchor the electrode 60 at a predetermined position after implantation, the electrode 60 may also be equipped with an anchor 601, which is spiral-shaped and gradually converges in outer diameter. When the electrode 60 is implanted, the anchor 601 can be inserted and fixed to surrounding tissues by rotating the electrode 60.

Referring to Fig. 21, in another embodiment, the anchor 601 is made of memory metal. When delivered inside the human body, the anchor 601 can be attached to the outer circumference of the electrode 60 under the restraint of an external tube. When it reaches the predetermined position and the restraint is released, the anchor 601 returns to its predetermined posture in the human body, i.e., flipping outward to form a barbed structure to anchor to the surrounding tissues.

Referring to Fig. 22, in another embodiment, another implantation pathway for the electrode 60 is provided, such as entering the inferior vena cava through surface puncture, and then entering the right atrium 11, and then passing through the interventricular septum and entering the left atrium, and then passing through the mitral valve and entering the left ventricle 1, and finally releasing the electrode 60 and anchoring it to the ventricular free wall 7. The number of the electrodes 60 may be single or multiple, and the implantation site may be in the ventricular free wall or the interventricular septum as described in the previous embodiments, wherein specific interventional delivery may be combined with the previous embodiments.

Referring to Fig. 23, in another embodiment, another implantation pathway for the electrode 60 is provided, such as entering the inferior vena cava or superior vena cava through surface puncture and then entering the right atrium 11, then passing through the tricuspid valve into the right ventricle 2, and finally releasing the electrode 60 and anchoring it to the ventricular free wall of the right ventricle 2. The number of electrodes 60 may be single or multiple, and the implantation site may be in the ventricular free wall or the interventricular septum as described in the previous embodiments, wherein specific interventional delivery may be combined with the previous embodiments.

The various technical features of the above embodiments can be combined in any way. In order to make the description concise, not all possible combinations of the technical features in the above embodiments have been described. However, as long as there is no contradiction in the combination of these technical features, they should be considered within the scope of this specification. When the technical features of different embodiments are shown in the same figure, it can be regarded that this figure discloses the combination of the embodiments involved.

The above embodiments only express several embodiments of the present application, and their descriptions are more specific and detailed, but should not be understood as limiting the scope of the patent application. It should be pointed out that for those ordinary skilled in the art, modifications and improvements can be made without departing from the concept of this application, which should be within the protection scope of this application.

## Claims

1. An implant delivery system comprising a sheath and an implant, wherein
the sheath comprises a hollow sheath body, the implant is releasably disposed within the sheath body, a distal end of the sheath body is a puncture tip capable of penetrating into a ventricular myocardium and guiding the sheath body to pass through the ventricular myocardium, and a length of the sheath body at least is sufficient to extend from outside a body, via a cardiac apex, into the ventricular myocardium.

2. The implant delivery system of claim 1, wherein a pushing device is slidably set inside the sheath body, and a distal end of the pushing device is capable of pushing the implant outside the sheath body for releasing; and wherein
the pushing device is tubular-shaped and has an internal threading channel, a proximal end of the implant is fixed with a connecting wire or provided with a detachable connecting wire, and the connecting wire extends through the threading channel towards a proximal end.

3. The implant delivery system of claim 1, wherein an internal of the sheath body comprises a first lumen and a second lumen that extend in parallel from a distal end to a proximal end, and the implant comprises multiple implants arranged along a length direction inside the first lumen;
a separating portion is provided between the first lumen and the second lumen, and multiple avoidance holes are defined in the separating portion; at least one of the avoidance holes is provided at a proximal end side of each implant, a connecting wire of each implant extend from a corresponding avoidance hole to the second lumen and then out of the proximal end of the sheath body.

4. The implant delivery system of claim 3, wherein damping structures acting with each other are provided between the first lumen and each implant.

5. The implant delivery system of claim 3, wherein a tubular-shaped pushing device is provided inside the second lumen for receiving the connecting wire.

6. The implant delivery system of claim 5, wherein the pushing device is movable along the connecting wire to enter the first lumen through the corresponding avoidance hole to push a corresponding implant, and the connecting wire is a conducting wire or a traction wire.

7. The implant delivery system of claim 1, wherein the implant is an implantable electrode, and the system further comprises a pulse generator, and wherein
the implantable electrode and the pulse generator are formed separately, and the implantable electrode has a smooth outer surface; or
the implantable electrode and the pulse generator are formed integrally and have a smooth outer surface.

8. The implant delivery system of claim 1, wherein the implant is an implantable electrode, and the system further comprises a pulse generator, the implantable electrode has an anchor configured for acting on surrounding tissues, and the implantable electrode and the pulse generator are formed separately or integrally.

9. The implant delivery system of claim 1, wherein the length of the sheath body is 12~20cm.

10. The implant delivery system of claim 1, wherein a steering wire extends movably through the sheath body, a distal end of the steering wire is connected to an inner wall of the puncture tip, and a proximal end of the steering wire extends out of the sheath body and acts as a force application end for pulling the puncture tip to change its orientation.

11. The implant delivery system of claim 10, wherein the system further comprises a fixed handle with a transitional lumen defined therein, the proximal end of the sheath body is inserted into the fixed handle in a sealed manner, an inner lumen of the sheath body communicates with the transitional lumen, an electrode input port is provided on the fixed handle and communicates with the transitional lumen, and the proximal end of the steering wire extends out of the fixed handle.

12. The implant delivery system of claim 11, wherein a branch pipe is provided on a side wall of the fixed handle and communicates with the transitional lumen, and the steering wire extends out of the fixed handle from the branch pipe.

13. A cardiac rhythm adjustment system comprising:
at least one electrode, capable of being implanted into a ventricular myocardium; and
a sheath comprising a hollow sheath body, wherein the at least one electrode is releasably disposed within the sheath body, a distal end of the sheath body is a puncture tip capable of penetrating into the ventricular myocardium and guiding the sheath body to pass through the ventricular myocardium, and a length of the sheath body at least is sufficient to extend from outside a body into the ventricular myocardium.

14. The cardiac rhythm adjustment system of claim 13, wherein the at least one electrode enters the ventricular myocardium through an epicardium.

15. The cardiac rhythm adjustment system of claim 13, wherein the at least one electrode enters the ventricular myocardium through an endocardium.

16. The cardiac rhythm adjustment system of claim 13, wherein an implantation site of the at least one electrode is a predetermined electrical stimulation position located in at least one of an interventricular septum myocardium, a left ventricular free wall myocardium, and a right ventricular free wall myocardium.

17. The cardiac rhythm adjustment system of claim 16, wherein the implantation site of the at least one electrode comprises multiple predetermined electrical stimulation positions, one of which is close to distal branches of a His bundle or the left bundle branch or the right bundle branch.

18. The cardiac rhythm adjustment system of claim 16, wherein the at least one electrode comprises one electrode or multiple electrodes implanted in the interventricular septum myocardium, and the multiple electrodes are arranged at intervals along a long axis of the interventricular septum.

19. The cardiac rhythm adjustment system of claim 16, wherein the at least one electrode comprises one electrode or multiple electrodes implanted in the ventricular free wall myocardium, and the multiple electrodes are arranged at intervals along a long axis of the ventricular free wall.

20. The cardiac rhythm adjustment system of claim 16, wherein the at least one electrode comprises a pacing electrode implanted in the interventricular septum myocardium, and a pacing electrode and/or a defibrillation electrode implanted in the ventricular free wall myocardium.

21. The cardiac rhythm adjustment system of claim 13, wherein the at least one electrode comprises one group or multiple groups of electrodes, and the electrodes of different groups enter an implantation pathway through a same puncture point.

22. The cardiac rhythm adjustment system of claim 13, wherein the at least one electrode comprises one group or multiple groups of electrodes, each group of electrodes comprise one or multiple electrodes, and the multiple electrodes of each group correspond to one path extending approximately in a straight line within the ventricular myocardium.

23. The cardiac rhythm adjustment system of claim 22, wherein the paths within the ventricular myocardium are distributed radially around a cardiac apex; or, the paths within the ventricular myocardium are parallel to each other and extend along a long axis of a heart.

24. The cardiac rhythm adjustment system of claim 13, further comprising a pulse generator connected to the at least one electrode through a circuit, and the at least one electrode and the pulse generator being formed separately or integrally.

25. The cardiac rhythm adjustment system of claim 13, further comprising a guiding device, wherein the guiding device comprises a base that is capable of being fixed to a body surface and an adjustable cooperating seat installed on the base, and the sheath is assembled to the cooperating seat.

26. The cardiac rhythm adjustment system of claim 25, wherein an arc-shaped guide rail is fixed on the base, the cooperating seat is installed on the arc-shaped guide rail in a slidable and positioning manner, and the sheath extends through the cooperating seat in a movable and positioning manner.

27. The cardiac rhythm adjustment system of claim 26, wherein a steering wire extends movably through the sheath body;
the arc-shaped guide rail determines a first plane, and the steering wire pulls the sheath to bend in a second plane at a distal end, wherein the first plane is perpendicular to the second plane.

28. The cardiac rhythm adjustment system of claim 25, further comprising an imaging system used to guide the sheath to puncture and display a position of the at least one electrode.

29. The cardiac rhythm adjustment system of claim 28, wherein an ultrasound probe is provided on the cooperating seat to collect the position of the at least one electrode and a posture of the sheath in real-time, which is simulated and displayed at a corresponding position of a pre-constructed heart model through the imaging system, for comparison with a predetermined implantation site.

30. The cardiac rhythm adjustment system of claim 13, wherein an implantation site of the at least one electrode is a predetermined electrical stimulation position, and the cardiac rhythm adjustment system further comprises a navigation simulation system for indication relative positions of the electrical stimulation position and the at least one electrode in real-time; and wherein
the navigation simulation system performs three-dimensional modeling of the heart to obtain the pre-constructed heart model, and collects the position of the at least one electrode in real-time through the imaging system and displays it in a corresponding position of the heart model during use, for comparison with a predetermined electrical stimulation position.

31. The cardiac rhythm adjustment system of claim 13, further comprising a pushing device, wherein the push device is tubular-shaped and has an internal threading channel, the at least one electrode is connected to a connecting wire which extends towards a proximal end through the threading channel.

32. The cardiac rhythm adjustment system of claim 13, wherein an internal of the sheath body comprises a first lumen and a second lumen that extend in parallel from a distal end to a proximal end, and the at least one electrode comprises multiple electrodes arranged along a length direction inside the first lumen; a separating portion is provided between the first lumen and the second lumen, and multiple avoidance holes are defined in the separating portion with at least one of the avoidance holes being provided at a proximal end side of each electrode, each electrode is connected to a connecting wire that extending from a corresponding avoidance hole to the second lumen and then out of the proximal end of the sheath body.

33. A cardiac rhythm adjustment method, comprising:
planning an implantation pathway;
providing a delivery system with pre-installed electrode and delivering the delivery system into a ventricular myocardium of a human heart along the implantation pathway; and
releasing the electrode.

34. The cardiac rhythm adjustment method of claim 33, wherein the delivery system comprises a sheath for loading the electrode, and delivering the electrode comprises puncturing into the implantation pathway through an epicardium until a distal end of the sheath reach a predetermined electrical stimulation position.

35. The cardiac rhythm adjustment method of claim 33, wherein the delivery system adopts sheath approach, and delivering the electrode comprises puncturing into a myocardium through an epicardium; and then
releasing the electrode and maintaining it at the predetermined electrical stimulation position to perform electrical stimulation.

36. The cardiac rhythm adjustment method of claim 33, wherein planning the implantation pathway comprises:
collecting basic data of the heart;
performing 3D-modeling of the heart to obtain a heart model, wherein collecting the basic data is based on one or multiple of three-dimensional ultrasound, CT, magnetic resonance imaging; and structures of an interventricular septum, a ventricular free wall, and an endocardium are displayed in the heart model; and
calculating the electrical stimulation position and planning the implantation pathway correspondingly.

37. The cardiac rhythm adjustment method of claim 33, wherein a distal end of the delivery system is a puncture tip.
